(19) Europäisches Patentamt
European Patent Office
Office européen des brevets

(11) **EP 1 037 048 A2**

(12) **EUROPEAN PATENT APPLICATION**

(43) Date of publication:
**20.09.2000 Bulletin 2000/38**

(51) Int. Cl.$^7$: **G01N 33/52**, C12Q 1/54,
C12Q 1/60

(21) Application number: **00104594.7**

(22) Date of filing: **15.03.2000**

(84) Designated Contracting States:
**AT BE CH CY DE DK ES FI FR GB GR IE IT LI LU MC NL PT SE**
Designated Extension States:
**AL LT LV MK RO SI**

(30) Priority: **15.03.1999 JP 6858999**

(71) Applicant:
**FUJI PHOTO FILM CO., LTD.**
**Kanagawa-ken, 250-0123 (JP)**

(72) Inventors:
• **Mori, Toshihiro**
**Asaka-shi, Saitama 351-8585 (JP)**
• **Arai, Takaki**
**Asaka-shi, Saitama 351-8585 (JP)**
• **Amano, Yoshikazu**
**Asaka-shi, Saitama 351-8585 (JP)**

(74) Representative:
**Weisert, Annekäte, Dipl.-Ing. Dr.-Ing. et al**
**Patentanwälte**
**Kraus & Weisert**
**Thomas-Wimmer-Ring 15**
**80539 München (DE)**

(54) **Quantitative analysis of glucose or cholesterol in whole blood**

(57)     Quantitative analysis of glucose or cholesterol in a whole blood sample employing a united multilayer analytical element which contains peroxidase, a dye-forming reagent composition, and glucose oxidase or cholesterol oxidase, is performed by comparing a concentration of glucose or cholesterol at an optionally predetermined hematocrit value with a calibration curve which indicates a relationship between a deviation of a hematocrit value from the predetermined hematocrit value and a deviation of a concentration of glucose or cholesterol.

EP 1 037 048 A2

**Description**

FIELD OF THE INVENTION

The present invention relates to a quantitative analysis of glucose or cholesterol in a whole blood sample employing a multilayer analytical element. In particular, the present invention relates to a method for quantitatively determining a concentration of glucose or cholesterol in a whole blood sample under the condition that the error originating from variation of a hematocrit value is easily and reliably corrected.

BACKGROUND OF THE INVENTION

The quantitative analysis of glucose or cholesterol in a whole blood sample gives useful information for diagnosis and treatment of various deceases. In the analysis of glucose or cholesterol, a dry analytical method utilizing an oxidase reaction system which produces hydrogen peroxide is generally employed. The dry analytical method is performed using a multilayer analytical element in the form of a film or a sheet which contains one or more reagents which directly or indirectly react with glucose or cholesterol to give color formation in the analytical element. The analytical element generally comprises a light-transmissive support, one or more reagent layers, and a spreading layer arranged in order. It is known that blood samples such as plasma, serum and whole blood have different hematocrit values (i.e., volume ratio (%) of erythrocyte in the blood sample), different protein concentrations, different viscosity values, different lipid values, and like. The quantitative analysis of an analyte in a blood sample is sometimes disturbed by variations of these various factors. Further, the quantitative analysis is affected by certain additives and the period of storage of blood samples.

It is further known that the analysis of an analyte in a whole blood sample is greatly affected by the hematocrit value. Particularly, the quantitative analysis of glucose or cholesterol in a whole blood sample is disturbed by variation of the hematocrit value.

The quantitative analysis of glucose or cholesterol is generally performed using a reagent composition which comprises peroxidase, a dye-forming reagent, and glucose oxidase or cholesterol oxidase. In the known analytical method for quantitative analysis of glucose or cholesterol in a blood sample, the blood sample is spotted onto a multilayer analytical element and then the resulting color formation is detected by a spectrophotometer. This analytical method is useful when the blood sample is plasma or serum which contains no erythrocyte. However, if a whole blood sample is employed, the red color of erythrocyte contained in the sample apparently disturbs the detection of the color produced by the reaction between the analyte (e.g., glucose or cholesterol) and the color-forming reagent composition in the analytical element.

It has been proposed to incorporate an erythrocyte-removing filter layer in the analytical element to separate the erythrocyte from the blood sample in the analytical element, so as to produce a colorless liquid sample in the analytical element. This procedure may partly solve the problem caused by the presence of red erythrocyte in the whole blood sample. However, the problem caused by variation of the hematocrit value which affects the quantitative analysis of an analyte such as glucose still remains.

Accordingly, it is required for the precise quantitative analysis of an analyte such as glucose or cholesterol to correct the analytically measured values by removing the error having been introduced by the variation of hematocrit value.

Japanese Patent Provisional Publication No. 59-108942 describes a method of quantitative analysis which comprises steps for correcting the analytical value by removing the error introduced by the variation of hematocrit value. There are described the following three alternative correcting processes:

(1) On a multilayer analytical sheet comprising a light-transmissive support, a reagent layer containing peroxidase and 4-aminoantipyrine/coupling agent, a light-shielding layer containing glucose oxidase, and a porous spreading layer comprising broadcloth, is spotted a whole blood sample, and color formation in the analytical element is detected at a wavelength of 550 nm from the support side. The primary glucose concentration is then obtained. Then, the optical density of red color of erythrocyte remaining on the spreading layer is measured at the same wavelength from the side of spreading layer, to give a correcting factor. The primary glucose concentration is corrected using the correcting factor to give a corrected glucose concentration.

(2) The primary glucose concentration is obtained in the manner described in (1) above. Separately, the area or spread circle of erythrocyte placed on the spreading layer is measured from the side of spreading layer to give a correcting factor. The primary glucose concentration is corrected using the correcting factor to give a corrected glucose concentration.

(3) The primary glucose concentration is obtained in the manner described in (1) above. Separately, the area or spread circle of erythrocyte placed on the spreading layer is measured from the support side to give a correcting factor. The primary glucose concentration is corrected using the correcting factor to give a corrected glucose concentration.

The analytical methods described above enable to give a reliable analytical value which is almost independent of variation of hematocrit value. However, there are certain drawbacks in these analytical methods. For instance, the provision of the light-shielding layer which serves as an erythrocyte-filtering layer disturbs rapid development of the analytical reaction and reduces the analytical accuracy. Further, so long as the analytical method of (1) above is concerned, the red color of erythrocyte disturbs the spectroscopic measurements of the formed color because the measurement of the formed color and the measurement of the erythrocyte on the spreading layer are performed at the same wavelength. As for the analytical methods of (2) and (3) above, it is not easy to accurately measure the area or spread circle of the erythrocyte remaining on the spreading layer.

Japanese Patent Provisional Publication No. 9-121894 describes a reagent strip for analysis of whole blood samples comprising an anisotropic membrane containing an acrylate polymer which is relatively insensitive to the hematocrit value in blood samples (namely, hematocrit value-adjusting agent). It is described that the reagent strip gives a glucose analytical value which is essentially independent of hematocrit values within a hematocrit range of approx. 20% to 60%. However, in order to prepare a reagent strip giving such accurate analytical value, the acrylate polymer is required to have a very high polymerization degree. Moreover, an acrylate polymer having a very high polymerization degree is sparingly soluble in a solvent, and therefore it is not easy to produce the reagent strip in industry.

Accordingly, the present invention has an object to provide a method for quantitatively analyzing a concentration of glucose or cholesterol in a whole blood sample using an easily available multilayer analytical element, to give an accurate analytical value regardless of variation of hematocrit value in the sample.

SUMMARY OF THE INVENTION

The present invention resides in a method for quantitatively analyzing a concentration of glucose or cholesterol in a whole blood sample employing a united multilayer analytical element which contains peroxidase, a dye-forming reagent composition, and glucose oxidase or cholesterol oxidase, comprising the steps of:

(1) applying the whole blood sample onto the analytical element and subsequently measuring an optical density of red color of erythrocyte developed in the analytical element spectroscopically at a wavelength predetermined in the region of 500 nm to 590 nm;
(2) comparing the optical density measured in the step (1) with a calibration curve which indicates a relationship between a hematocrit value and an optical density at the predetermined wavelength and which is prepared using an equivalent united multilayer analytical element, so as to determine a hematocrit value of the whole blood sample;
(3) measuring an optical density of color of erythrocyte developed in the analytical element at a predetermined wavelength of shorter than 500 nm or longer than 590 nm;
(4) comparing the optical density measured in the step (3) with a calibration curve which indicates a relationship between a concentration of glucose or cholesterol and an optical density at the wavelength adopted in the step (3) at a predetermined hematocrit value, to determine a concentration of glucose or cholesterol under the assumption that the whole blood sample has the predetermined hematocrit value;
and
(5) comparing the concentration of glucose or cholesterol determined in the step (4) under the assumption that the whole blood sample has the predetermined hematocrit value and the hematocrit value of the whole blood sample determined in the step (2), with a separately prepared calibration curve which indicates a relationship between a deviation of a hematocrit value from the predetermined hematocrit value adapted in the step (4) and a deviation of a concentration of glucose or cholesterol, to determine the concentration of glucose or cholesterol in the whole blood sample.

BRIEF DESCRIPTION OF DRAWINGS

Fig. 1 schematically illustrates a multilayer analytical element (A) which is favorably employable in the analytical method of the invention.
Fig. 2 schematically illustrates a multilayer analytical element (B) which is also favorably employable in the analytical method of the invention.
Fig. 3 schematically illustrates a multilayer analytical element (C) which is also favorably employable in the analytical method of the invention.

Fig. 4 illustrates a calibration curve indicating a relationship between a glucose concentration in a whole blood sample and a color formation under the condition that the hematocrit value is 40 vol.%.

Fig. 5 illustrates a calibration curve indicating a relationship between a red color and a hematocrit value.

Fig. 6 illustrates a calibration curve indicating a relationship between a hematocrit value and a deviation.

Fig. 7 graphically illustrates the results of Example 1 and Comparison Example 1.

DETAILED DESCRIPTION OF THE INVENTION

Preferred embodiments of the analytical method according to the invention are described below:

1) The calibration curve employed in the step (2) is prepared using three or more whole blood samples which are known in their hematocrit values and differ from each other in the hematocrit values.

2) The wavelength adopted in the step (3) is in the region of 600 nm to 700 nm.

3) The calibration curve employed in the step (4) is prepared using three or more whole blood samples which all have the predetermined hematocrit value but differ from each other in the concentration of glucose or cholesterol.

4) The calibration curve employed in the step (5) is prepared using three or more whole blood samples which all have the same concentration of glucose or cholesterol but differ from each other in their hematocrit values.

5) The analytical element employed in the analytical method comprises a porous reagent sheet which has a void volume of 30% to 80% and contains peroxidase, a dye-forming reagent composition, and glucose oxidase or cholesterol oxidase.

6) The analytical element employed in the analytical method comprises a light-transmissive support, an adhesive layer, and a porous reagent layer which has a void volume of 30% to 80% and contains peroxidase, a dye-forming reagent composition, and glucose oxidase or cholesterol oxidase.

7) The analytical element employed in the analytical method comprises a light-transmissive support, a reagent layer containing peroxidase and a dye-forming reagent composition, and a porous reagent layer which has a void volume of 30% to 80% and contains glucose oxidase or cholesterol oxidase.

8) The analytical element employed in the analytical method has no light-shielding layer.

The analytical method of the invention is characteristic in that the analytical error occurring in the analysis of glucose or cholesterol in a whole blood sample which is brought by the presence of erythrocyte and variation of a hematocrit value in the whole blood sample is removed not by the separation of erythrocyte using a filter, the provision of a light-shielding layer, and/or the measurement of the amount of erythrocyte, but by measuring separately the color produced in the analytical element by the reaction between glucose or cholesterol and the dye-forming reagent composition (i.e., color-producing compound or composition) and the red color of erythrocyte, using lights having different wavelengths for the measurement. The value measured on the produced color is corrected by the use of the value measured on the red color of erythrocyte.

The analytical method of the invention is further described below in more detail. In the following description, a glucose concentration or a cholesterol concentration which is not corrected with respect to the deviation introduced by the variation of hematocrit value is referred to as "glucose concentration (uncorrected)" or "cholesterol concentration (uncorrected)", respectively. A glucose concentration or a cholesterol concentration which is produced by incorporating the correction is referred to as "glucose concentration (corrected)" or "cholesterol concentration (corrected)", respectively.

In the first step, the whole blood sample is spotted on the analytical element and subsequently an optical density of red color of erythrocyte developed in the analytical element is spectroscopically measured at a wavelength which is predetermined in the region of 500 nm to 590 nm. The whole blood sample is spotted, preferably, in an amount of 1 to 10 μL. The analytical element having the spotted whole blood sample may be incubated, if necessary. The spectroscopic measurement is performed by detecting a light reflected on the analytical element in the manner, for instance, described in Japanese Patent Provisional Publication No. 59-108942. The red color of erythrocyte can be measured by an end point method or a reaction rate method. The end point method is preferably employed.

In the second step, the optical density measured in the initial step [namely, step (1)] is compared with a calibration curve [Calibration Curve (I)] which indicates a relationship between a hematocrit value and an optical density at the predetermined wavelength. The calibration curve is prepared using an equivalent united multilayer analytical element, so as to determine a hematocrit value of the whole blood sample. This calibration curve can be prepared preferably using three or more whole blood samples having different hematocrit values which are already known. The calibration curve may be prepared using two whole blood samples.

In the third step, an optical density of color of erythrocyte developed in the analytical element after the spotting of the whole blood sample is spectroscopically measured at a predetermined wavelength of shorter than 500 nm or longer than 590 nm. If necessary, an incubation step is added. The spectroscopic measurement is preferably performed at a wavelength between 600 nm to 700 nm, utilizing an end point method.

In the fourth step, the optical density measured in the third step [namely, step (3)] is compared with a calibration curve [Calibration Curve (II)] which indicates a relationship between a concentration of glucose or cholesterol and an optical density at the wavelength adopted in the third step at a predetermined hematocrit value, to determine a concentration of glucose or cholesterol [that is, a glucose concentration (uncorrected) or a cholesterol concentration (uncorrected)] under the assumption that the whole blood sample has the predetermined hematocrit value. The glucose concentration (uncorrected) or cholesterol concentration (uncorrected) represents a value determined under the assumption that a hematocrit value of the whole blood sample is equal to the predetermined hematocrit value. Accordingly, the glucose concentration (uncorrected) or cholesterol concentration (uncorrected) is not free from the deviation caused by variation of the hematocrit value. Any predetermined hematocrit value other than 0% is utilized in the fourth step.

In the fifth step, the glucose concentration (corrected) or cholesterol concentration (corrected) is obtained utilizing a relationship between the concentration of glucose or cholesterol [that is, a glucose concentration (uncorrected) or a cholesterol concentration (uncorrected)] and the hematocrit value of the whole blood sample determined in the second step. As described above, the glucose concentration (uncorrected) or cholesterol concentration (uncorrected) represents a value determined under the assumption that a hematocrit value of the whole blood sample is equal to the predetermined hematocrit value. Accordingly, the glucose concentration (uncorrected) or cholesterol concentration (uncorrected) is deviated from the real value (namely, corrected value). The deviation is adjusted by comparing the uncorrected value and the hematocrit value determined on the whole blood sample with a calibration curve [Calibration curve (III)] which indicates a relationship between a deviation of a hematocrit value from the predetermined hematocrit value adopted in the fourth step and a deviation of a concentration of glucose or cholesterol. Calibration curve (III) can be separately prepared, for instance, utilizing three or more whole blood samples which have different hematocrit values which are already known.

Thus, the desired glucose concentration (corrected) or cholesterol concentration (corrected) is determined.

In the above-mentioned steps, the first step and the third step can be performed in parallel or the third step is performed in advance of the first step.

The comparison of the analytical values with the calibration curve in the above-mentioned steps can be conducted in a computerized dry analytical apparatus.

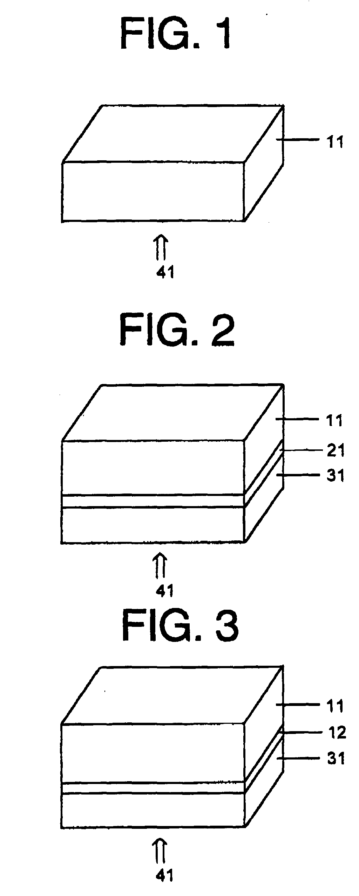

The analytical method of the invention can be favorably performed utilizing various united multilayer analytical elements, such as, those illustrated in Figs. 1 to 3.

The analytical element A illustrated in Fig. 1 comprises a porous reagent sheet (11) which has a void volume of 30% to 80% and contains peroxidase, a dye-forming reagent composition, and glucose oxidase or cholesterol oxidase.

The analytical element B illustrated in Fig. 2 comprises a light-transmissive support (31), an adhesive layer (21) , and a porous reagent layer (11) which has a void volume of 30% to 80% and contains peroxidase, a dye-forming reagent composition, and glucose oxidase or cholesterol oxidase. The light-transmissive support (31) is provided to the analytical element so as to easily handle the analytical element.

The analytical element C illustrated in Fig. 3 comprises a light-transmissive support (31), a reagent layer (12) containing peroxidase and a dye-forming reagent composition, and a porous reagent layer (11) which has a void volume of 30% to 80% and contains glucose oxidase or cholesterol oxidase. The light-transmissive support (31) is provided to the analytical element so as to easily handle the analytical element.

The analytical element employed in the analytical method of the invention preferably has no light-shielding layer.

The spectroscopic measurement of the red color and the color produced in the analytical element is preferably performed from the support side in the case that the analytical elements B and C are employed. In the case of using the analytical element A, the spectroscopic measurement can be performed from either side.

The analytical elements A and B are further described below.

Each of the porous reagent sheet of the analytical element A and the porous reagent layer of the analytical elements B comprises porous material which has a void volume of 30% to 80% and contains a dye-forming reagent composition comprising peroxidase, a dye-forming reagent composition, and glucose oxidase or cholesterol oxidase. The dye-producing reagent composition can be incorporated into the porous material by coating an aqueous solution of the color-producing reagent composition on the porous material or dipping the porous material into the aqueous solution.

The porous reagent sheet or layer preferably has a thickness of 1 to 500 µm, more preferably 5 to 300 µm.

The porous material having a void volume of 30% to 80% can be a fabric sheet or a non-fabric sheet. The fabric sheet can be a sheet of textile fabric, knitted fabric, non-woven fabric, filter paper, or agglomerated short fibers. The non-fabric sheet typically is a membrane filter. A sheet of diatomaceous particles dispersed in a binder may be employed. A sheet of glass beads or resin particles bonded together by adhesive is also employable. Preferred is a fabric sheet having a void volume of 30 to 80%, preferably 50% to 70%. A knitted fabric is most preferred.

The knitted fabric can be plain knitted fabric, tricot knitted fabric, double tricot knitted fabric, or milanese tricot fabric. The fibrous material tar forming the knitted fabrics can be of natural origin such as cotton, kapok, flax, hemp, ramie, silk or wool, or of synthetic origin such as polyamide, polyester, rayon, cupra, cellulose acetate, polyvinyl alcohol, polyacrylate, or polyethylene terephthalate. These fibrous materials can be employed in combination. The thickness of the fibrous material preferably is in the range of approx. 20 to 300, more preferably approx. 40 to 130 denier. The fibrous material preferably is a filament fiber or spun fibers. The spun fibers are preferred. The knitted fabric preferably has a thickness of approx. 100 to 500 μm, more preferably approx. 120 to 350 μm.

Glucose oxidase preferably originates from *Aspergillus niger* or *Penicillium notatum.*

Cholesterol oxidase preferably originates from Nocardia erythroporis, *Brevibacterium*, *Pseudomonas, Mycobacterium*, or mushrooms.

Each of glucose oxidase and cholesterol oxidase is preferably contained in the porous reagent sheet or layer in an amount of 2,000 to 40,000 $U/m^2$, more preferably 4,000 to 30,000 $U/m^2$.

Peroxidase can be of plant origin, animal origin, or microorganism origin. Preferred are peroxidases of plant origin and microorganism origin. Most preferred are peroxidase extracted from horseradish or Japanese radish, and peroxidase extracted from microorganisms of *genus Cochliobolus* or *genus Curvularia*.

Peroxidase is preferably contained in the porous reagent layer in an amount of 5,000 to 100,000 $U/m^2$, more preferably 10,000 to 60,000 $U/m^2$.

There is no specific limitation with respect to the dye-forming compound, provided that it gives a dye under hydrogen peroxide in the presence of an oxidative catalyst such as peroxidase. Leuco dyes or a dye-forming reagent composition comprising 4-aminoantipyrine (4-AAP) and a coupling reagent which is coupled with 4-AAP.

The leuco dye produces coloring under catalytic action of an oxidizing compound such as peroxidase in the presence of hydrogen peroxide. Examples of the leuco dyes are described in Japanese Patent Provisional Publication No. 59-193352. The leuco dyes described in the publication produce blue color having an absorption peak of 600 nm or longer, specifically in the region of 600 to 700 nm. Preferred leuco dyes are 2-(3,5-dimethoxy-4-hydroxyphenyl)-4-[4-(dimethylamino)phenyl]-5-phenethylimidazole, 2-(3,5-dimethoxy-4-hydroxyphenyl)-4-[4-(dimethylamino)phenyl]-5-benzylimidazole, and their salts. Particularly preferred is acetate of 2-(3,5-dimethoxy-4-hydroxyphenyl)-4-[4-(dimethylamino)phenyl]-5-phenethylimidazole.

The dye-forming reagent composition comprising 4-aminoantipyrine (4-AAP) and a coupling reagent which is coupled with 4-AAP preferably a dye having a blue color which corresponds an absorption peak of a wavelength of longer than 600 nm, particularly 600 nm to 700 nm. The color can be varied depending on the coupling agent. Examples of the coupling agents which give a preferred color in the above-mentioned wavelength region include phenols (e.g., p-chlorophenol), naphthalene derivatives (e.g., 1,7-dihydronaphthalene), toluidine derivatives (e.g., N-ethyl-N-2-sulfoethyl-m-toluidine, and N,N-diethyl-m-toluidine), dihydroindoles, tetrahydroquinolines, and substituted aniline compounds (e.g, 5,6,7,8-tetrahydro-1-naphthylamine). Particularly preferred is 8-anilino-1-naphthalenesulfonic acid.

The dye-forming reagent composition is preferably contained in the porous reagent sheet or layer in an amount of 0.1 to 10 $g/m^2$, more preferably 1 to 10 $g/m^2$.

The porous sheet or layer further can contain other additives such as a pH-adjusting agent and a surface active agents.

The pH-adjusting agent is selected to maintain an optimum pH condition for the enzyme composition (a combination of oxidase and peroxidase). Preferred pH-adjusting agents are phosphate buffers, citrate buffers, borate buffers, Tris buffer, and Good's buffer.

The surface active agents functions to make the porous reagent layer hydrophilic so as to accelerate the reaction between the leuco dye and hydrogen peroxide in the layer. Examples of the surface active agents are water-soluble nonionic, cationic, anionic, and amphoteric surface active agents. Examples of the nonionic surface active agents include dimethylcyclohexane/methyl(polyoxyethylene) copolymer, alkylaryl ethers of polyoxyethylene or polyglycerol, and fatty acid esters. A nonionic surface active agent having 8 to 15 oxyethylene or oxypropylene units is most preferred.

The analytical element B is prepared by combining the porous reagent sheet or layer and a light-transmissive support via an adhesive layer. The adhesive layer may be composed of a locally placed hot melt adhesive or a wholly placed hydrophilic adhesive.

Examples of the hot-melt adhesive include a solid adhesive (i.e., solid at room temperature) comprising thermoplastic resin. The hot-melt adhesive is melted in a heated applicator and spotted on the support sheet in the form of dots, lines, or their combinations. Thus formed non-continuous adhesive layer serves to efficiently supply airy oxygen into the porous reagent layer. The adhesive layer preferably has a thickness in the range of 5 to 100 μm, more preferably 10 to 30 μm. Examples of the hydrophilic adhesives include gelatin, gelatin derivatives, pullulan, pullulan derivatives, agarose, polyvinyl alcohol, polyvinyl pyrrolidone, and polyacrylamide. Gelatin is preferred. The hydraphilic adhesive layer is preferably coated with a surface active agent.

In not only the analytical element A but also the analytical element B, the spectroscopic measurement of the produced dye is directly done on the porous reagent sheet or layer.

The light-transmissive support is a non-water permissible transparent sheet. The light-transmissive support employable in the analytical element of the invention may be one of those described in Japanese Patent Provisional Publication No. 55-164356. Examples of the materials for producing the support are hydrophobic or nearly hydrophobic polymers such as polyethylene terephthalate, cellulose acetate, polycarbonate of bisphenol A, polystyrene, and polymethyl methacrylate. A transparent glass plate is also employable. Preferred is a polyethylene terephthalate sheet. The support preferably has a thickness of 50 $\mu$m to 1 mm, more preferably 80 to 400 $\mu$m, in the case of a polymer sheet, while it preferably has a thickness of 100 $\mu$m to 2 mm, more preferably 150 $\mu$m to 1 mm, in the case of a glass plate.

The surface of the support can be treated by known physical or chemical surface activating treating methods such as treatments with ultraviolet rays, corona discharge, and glow discharge, so as to have increased adhesion with the adhesive layer. Further, in addition to the physical or chemical surface activation or independently, a gelatin-subbing layer can be placed on the support.

The analytical element C is described below.

The analytical element comprises a light-transmissive support, a reagent layer containing peroxidase and a dye-forming reagent composition, and a porous reagent layer, which preferably comprises knitted fabric, which has a void volume of 30% to 80%, preferably 50% to 70%, and contains glucose oxidase or cholesterol oxidase. The porous reagent layer preferably has a thickness of 1 to 500 $\mu$m, more preferably 5 to 300 $\mu$m.

The reagent layer preferably comprises a hydrophilic polymer binder layer in which peroxidase and a dye-forming reagent composition are dispersed. The polymer binder preferably is one of the aforementioned hydrophilic polymers. Gelatin is most preferred. The reagent layer preferably has a thickness of 5 to 25 $\mu$m. The dye-forming reagent composition is preferably contained in the reagent layer in an amount of 0.1 to 1 g/m$^2$.

The analytical element of the invention is generally held in a mount (i.e., holder) in the manner described in Japanese Patent Provisional Publication No. 57-63452. The mount may be produced from polyethylene, polypropylene, polyvinyl chloride, polystyrene, polyamide, polycarbonate, polyethylene terephthalate, ABS resin, polymethyl methacrylate (PMMA) resin, ceramics (e.g., alumina and silica), or metals (e.g., aluminum, stainless steel, or titanium).

The analytical method of the invention is favorably employable particularly in the analysis of a whole blood sample containing glucose or cholesterol in an amount of 20 to 600 mg/dL. The analytical method of the invention can be employable in the analysis of a whole blood sample having hematocrit value at any level. However, a preferably employable whole blood sample has a hematocrit value of 20 to 60 vol.%.

The present invention is further described by the following examples.

Example 1

(1) Preparation of analytical element

An aqueous solution having the below-mentioned composition was coated on a colorless transparent polyethylene terephthalate film (thickness: 180 $\mu$m) in such amount that each component would be coated in the indicated amount.

| | |
|---|---|
| Alkali-treated gelatin | 14.5 g/m$^2$ |
| Surface active agent | 0.2 g/m$^2$ |
| Peroxidase | 10,000 U/m$^2$ |
| Leuco dye | 1 g/m$^2$ |
| 2-Morpholinoethanesulfonic acid (MES) | 0.2 g/m$^2$ |

The surface active agent was nonylphenoxypolyethoxyethanol (number of oxyethylene unit: average 9 to 10) [Surfactant 10G, available from Orion Co., Ltd.]. The leuco dye was 2-(3,5-dimethoxy-4-hydroxyphenyl)-4-[4-(dimethylamino)phenyl]-5-phenethylimidazole acetate.

The coated solution was dried at 50°C to give a reagent layer of 19 $\mu$m thick.

On the dry reagent layer were placed nonylphenoxypolyethoxyethanol (number of oxyethylene unit: average 9 to 10) and ion-exchanged water in an amount of 0.15 g/m$^2$ and 30 g/m$^2$, respectively, so that the reagent layer would swell.

On the swollen reagent layer was placed under light pressure a tricot knitted fabric (void volume: 60%, thickness: approx. 250 μm, produced by knitting polyethylene terephthalate yarn of 50 denier to give 36 gauge structure). Subsequently, on the knitted fabric was coated an aqueous solution of glucose oxidase (30,000 U/m$^2$) and 2-morpholinoethanesulfonic acid (MES, 1 g/m$^2$). The coated fabric was dried at 50°C to give a porous reagent sheet.

Thus, an analytical element having the structure of the analytical element C (Fig. 3) was prepared.

(2) Manufacture of analytical slide

The analytical element was cut to give square chips (12 mm x 13 mm) and mounted in a slide frame (described in Japanese Patent Provisional Publication No. 57-63452), to give a GLU-W dry analytical slide for analyzing a whole blood sample to quantitatively measure the amount of glucose.

(3) Preparation of calibration curve indicating relationship between glucose concentration and color formation

1) Preparation of blood sample

A fresh blood taken from a human body in the presence of sodium fluoride was centrifuged at 3,000 rpm to separate the blood into a plasma component and a blood cell component. Both components were mixed at an appropriate ratio to give a blood sample having a hematocrit value of 40 vol.%. The glucose concentration of the produced blood sample was measured by a hexokinase method to give a concentration of 100 mg/dL. The blood sample was divided into 7 sample portions. The 7 sample portions were adjusted in their glucose concentrations by the following methods. A 50 vol.% aqueous glucose solution (available from Otsuka Pharmaceutical Co., Ltd.) was added to give a sample portion having a higher glucose concentration. A sample portion is centrifuged and a portion of the resulting supernatant was removed and replaced with an ion-exchanged water to give a sample portion having a lower glucose concentration but keeping a hematocrit value of 40 vol.%. Thus, whole blood samples having a hematocrit value of 40 vol.% and a glucose concentration of 59, 100, 184, 295, 419, 520, or 500 mg/L were prepared.

2) Measurement of produced color

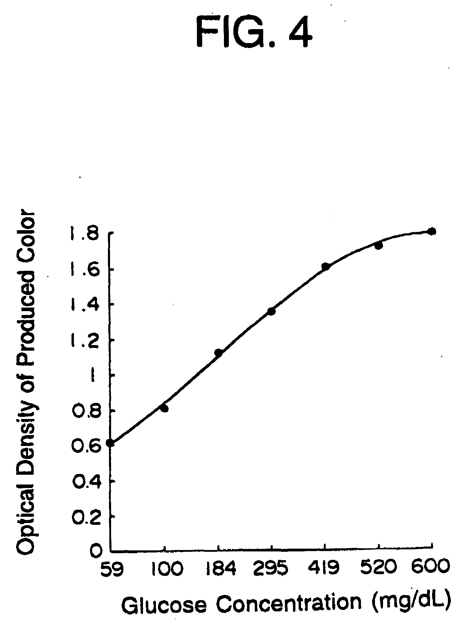

Each whole blood sample was spotted onto the GLU-W analytical element in an amount of 6 μL. At 60 seconds after the spotting, an optical density of a color produced in the analytical element was measured at 650 nm by means of a Fuji Drychem 5500 (available from Fuji Photo Film Co., Ltd.), to prepare a calibration curve indicating a relationship between glucose concentration and color optical density in the whole blood samples having a hematocrit value of 40 vol.%. Thus prepared calibration curve is illustrated in Fig. 4. The calibration curve can be expressed by the following equation (1):

$$Y = 922.63x(X)^3 - 1626x(X)^2 + 1247x(X) - 269.9 \qquad (1)$$

in which X represents a glucose concentration (mg/dL) and Y represents an optical density of produced color.

(4) Preparation of calibration curve indicating relationship between optical density of red color and hematocrit value

1) Preparation of blood sample

A fresh blood taken from a human body in the presence of heparin was centrifuged at 3,000 rpm to separate the blood into a plasma component and a blood cell component. Both components were mixed at an appropriate ratio to give blood samples having a hematocrit value of 25 vol.%, 40 vol.%, and 57 vol.%.

2) Measurement of optical density of red color

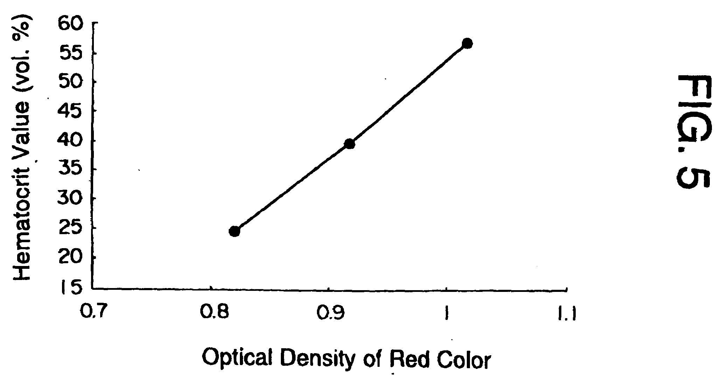

Each blood sample was spotted onto the GLU-W analytical element in an amount of 6 μL. At 30 seconds after the spotting, an optical density of a red color (red color of erythrocyte) was measured at 505 nm, to prepare a calibration curve indicating a relationship between hematocrit value and optical density of red color. Thus prepared calibration curve is illustrated in Fig. 5. The calibration curve can be expressed by the following equation (2):

$$Q = 162.28x(T) - 108.42 \qquad (2)$$

in which Q represents a hematocrit value (vol.%) and T represents an optical density of the red color of erythrocyte).

(4) Preparation of calibration curve indicating relationship between hematocrit value and deviation of glucose concentration caused by variation of hematocrit value

On the GLU-W analytical element was spotted 6 μL of each of the above-prepared three blood samples having different known hematocrit values and having the same glucose concentration. At 60 seconds after the spotting, an optical density of a color produced in the analytical element was measured at 650 nm. The optical density measured in the procedure was incorporated into the aforementioned equation (1) to obtain a glucose concentration (uncorrected) which is determined under the assumption that the hematocrit value was 40 vol.%. Thus obtained glucose concentration (uncorrected) was divided by a glucose concentration (uncorrected) having a hematocrit value of 40 vol.%, to obtain a deviation value (or ratio, in terms of %) representing the deviation of the glucose concentration (uncorrected) brought about by the deviation of hematocrit value. The results are set forth in Table 1.

Table 1

| Hematocrit value (vol.%) | Optical density of produced color | Glucose conc. (uncorrected) (mg/dL) | Deviation value (%) |
|---|---|---|---|
| 25 | 1.0725 | 336 | 115 |
| 40 | 1.0217 | 291 | 100 |
| 57 | 0.9656 | 249 | 85.6 |

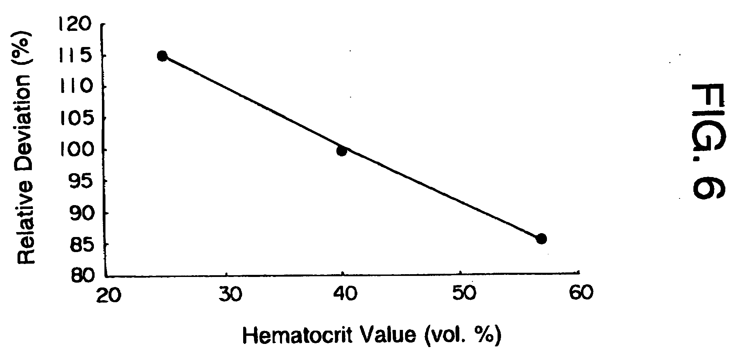

From the data set forth in Table 1, a calibration curve indicating a relationship between hematocrit value and deviation value was prepared. The prepared calibration curve is illustrated in Fig. 6. The calibration curve can be expressed by the following equation (3):

$$R = -0.9172 \times (Q) + 137.5 \qquad (3)$$

in which Q represents a hematocrit value (vol.%) and R represents a deviation value (%).

(5) Correction of glucose concentration in whole blood sample

1) A fresh blood taken from a human body in the presence of sodium fluoride was processed to give whole blood samples having a hematocrit value of 20 vol.%, 40 vol.% or 55 vol.%. The hematocrit values were determined by a capillary tube method. The glucose concentration in the whole blood sample was determined by a hexokinase method. It was confirmed that all blood samples had a glucose concentration of 100 mg/dL. The whole blood sample were named Samples A, B and C, in order.
2) Measurements of optical density values of red color and produced color
Each sample was spotted onto the GLU-W analytical element in an amount of 6 μL. At 30 seconds after the spotting, an optical density of a red color (t) was measured at 505 nm, and at 60 seconds after the spotting, an optical density (x) of a color produced in the analytical element was measured at 650 nm by means of a Fuji Drychem 5500 (available from Fuji Photo Film Co., Ltd.).
3) Calculations
The optical density (x) of produced color was incorporated into the aforementioned equation (1) to obtain a glucose concentration (y, uncorrected) in the sample under the assumption that the hematocrit value was 40 vol.%. The optical density (t) of red color was incorporated into the aforementioned equation (2) to obtain the actual hematocrit value (q).

The results are set forth in Table 2.

Table 2

| Sample | x | t | y (mg/dL) | q(vol.%) |
|---|---|---|---|---|
| A | 0.6903 | 0.8112 | 118 | 23.20 |

Table 2 (continued)

| Sample | x | t | y (mg/dL) | q(vol.%) |
|--------|--------|-------|-----------|----------|
| B | 0.6292 | 0.923 | 101 | 41.40 |
| C | 0.5788 | 0.998 | 88 | 53.50 |

The above-mentioned value for (q) was incorporated into the aforementioned equation (3) to obtain a deviation value (r) corresponding to the (q). The glucose concentration (uncorrected) in a whole blood sample, which is set forth in Table 2 and which is given under the assumption that the hematocrit value was 40 vol.%, was divided by the corresponding deviation value (in terms of ratio) to obtain a glucose concentration (corrected) in each sample at its actual hematocrit value. The results are set forth in Table 3.

Table 3

| Sample | r (%) | Glucose concentration (corrected) (mg/dL) |
|--------|--------|-------------------------------------------|
| A | 116.20 | 101.5 |
| B | 99.50 | 101.5 |
| C | 88.40 | 99.5 |

Comparison Example 1

The three blood samples (Samples A, B, and C) which were the same as those in the procedure for correction of glucose concentration in whole blood sample were employed in the comparison example.

Each sample was spotted onto the GLU-W analytical element in an amount of 6 $\mu$L. At 60 seconds after the spotting, an optical density ($x_1$) of a color produced in the analytical element was measured at 650 nm. Each of the measured optical density values was incorporated into the aforementioned equation (1) to obtain a glucose concentration ($y_1$) in the whole blood sample under the assumption that the hematocrit value was 40 vol.%.

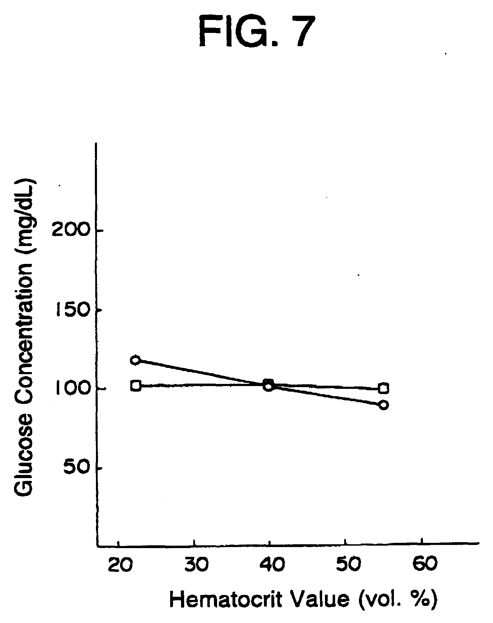

The results are set forth in Table 4 and illustrated in Fig. 7. In Fig. 7, the glucose concentration of each sample is expressed by -O-.

Table 4

| Sample | $x_1$ | $y_1$ (mg/dL) |
|--------|--------|---------------|
| A | 0.6903 | 118 |
| B | 0.6292 | 101 |
| C | 0.5788 | 88 |

From the results set forth in Table 4 and illustrated in Fig. 7, the glucose concentration obtained without correcting the error caused by variation of hematocrit value is apt to decrease when the hematocrit value increases. In contrast, the correction is effective to give a reliable glucose concentration in a whole blood sample even if the hematocrit value apparently varies.

**Claims**

1. A method for quantitatively analyzing a concentration of glucose or cholesterol in a whole blood sample employing a united multilayer analytical element which contains peroxidase, a dye-forming reagent composition, and glucose oxidase or cholesterol oxidase, comprising the steps of:

   (1) applying the whole blood sample onto the analytical element and subsequently measuring an optical density of red color of erythrocyte developed in the analytical element spectroscopically at a wavelength predetermined in the region of 500 nm to 590 nm;
   (2) comparing the optical density measured in the step (1) with a calibration curve which indicates a relationship between a hematocrit value and an optical density at the predetermined wavelength and which is prepared using an equivalent united multilayer analytical element, so as to determine a hematocrit value of the whole blood sample;

(3) measuring an optical density of color of erythrocyte developed in the analytical element at a predetermined wavelength of shorter than 500 nm or longer than 590 nm;

(4) comparing the optical density measured in the step (3) with a calibration curve which indicates a relationship between a concentration of glucose or cholesterol and an optical density at the wavelength adopted in the step (3) at a predetermined hematocrit value, to determine a concentration of glucose or cholesterol under the assumption that the whole blood sample has the predetermined hematocrit value;

and

(5) comparing the concentration of glucose or cholesterol determined in the step (4) under the assumption that the whole blood sample has the predetermined hematocrit value and the hematocrit value of the whole blood sample determined in the step (2), with a separately prepared calibration curve which indicates a relationship between a deviation of a hematocrit value from the predetermined hematocrit value adopted in the step (4) and a deviation of a concentration of glucose or cholesterol, to determine the concentration of glucose or cholesterol in the whole blood sample.

2.  The method of claim 1, wherein the calibration curve employed in the step (2) is prepared using three or more whole blood samples which are known in their hematocrit values and differ from each other in the hematocrit values.

3.  The method of claim 1, wherein the wavelength adopted in the step (3) is in the region of 600 nm to 700 nm.

4.  The method of claim 1, wherein the calibration curve employed in the step (4) is prepared using three or more whole blood samples which all have the predetermined hematocrit value but differ from each other in the concentration of glucose or cholesterol.

5.  The method of claim 1, wherein the calibration curve employed in the step (5) is prepared using three or more whole blood samples which all have the same concentration of glucose or cholesterol but differ from each other in their hematocrit values.

6.  The method of claim 1, in which the analytical element comprises a porous reagent sheet which has a void volume of 30% to 80% and contains peroxidase, a dye-forming reagent composition, and glucose oxidase or cholesterol oxidase.

7.  The method of claim 1, in which the analytical element comprises a light-transmissive support, an adhesive layer, and a porous reagent layer which has a void volume of 30% to 80% and contains peroxidase, a dye-forming reagent composition, and glucose oxidase or cholesterol oxidase.

8.  The method of claim 1, in which the analytical element comprises a light-transmissive support, a reagent layer containing peroxidase and a dye-forming reagent composition, and a porous reagent layer which has a void volume of 30% to 80% and contains glucose oxidase or cholesterol oxidase.

9.  The method of claim 1, in which the analytical element has no light-shielding layer.

# FIG. 1

11

41

# FIG. 2

11
21
31

41

# FIG. 3

11
12
31

41

# FIG. 4

# FIG. 5

# FIG. 6

# FIG. 7